(19) Europäisches Patentamt
European Patent Office
Office européen des brevets

(11) EP 4 349 339 A1

(12) EUROPEAN PATENT APPLICATION
published in accordance with Art. 153(4) EPC

(43) Date of publication:
**10.04.2024 Bulletin 2024/15**

(21) Application number: **22810554.0**

(22) Date of filing: **24.05.2022**

(51) International Patent Classification (IPC):
***A61K 31/506*** *(2006.01)* ***A61K 31/4439*** *(2006.01)*
***A61K 31/5377*** *(2006.01)* ***A61K 31/496*** *(2006.01)*
***A61K 31/519*** *(2006.01)* ***A61K 31/53*** *(2006.01)*
***A61P 35/00*** *(2006.01)* ***A61P 35/02*** *(2006.01)*

(52) Cooperative Patent Classification (CPC):
**A61K 31/395; A61K 31/4439; A61K 31/496; A61K 31/505; A61K 31/506; A61K 31/519; A61K 31/53; A61K 31/5377; A61K 45/06; A61P 35/00; A61P 35/02; A61P 35/04**

(86) International application number:
**PCT/CN2022/094725**

(87) International publication number:
**WO 2022/247829 (01.12.2022 Gazette 2022/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.05.2021 CN 202110572563**

(71) Applicant: **Hinova Pharmaceuticals Inc.**
**Chengdu, Sichuan 610041 (CN)**

(72) Inventors:
- **LI, Jing**
  **Chengdu, Sichuan 610041 (CN)**
- **DU, Wu**
  **Chengdu, Sichuan 610041 (CN)**
- **HUO, Yongxu**
  **Chengdu, Sichuan 610041 (CN)**
- **LI, Xinghai**
  **Chengdu, Sichuan 610041 (CN)**

(74) Representative: **Altmann Stößel Dick Patentanwälte PartG mbB**
**Theodor-Heuss-Anlage 2**
**68165 Mannheim (DE)**

# (54) PHARMACEUTICAL COMPOSITION FOR TREATMENT OF CANCER AND USE THEREOF

(57) A pharmaceutical composition for the treatment of cancer and a use thereof, belonging to the field of medicine. The pharmaceutical composition consists of an FAK inhibitor and a CD44 inhibitor. Also provided is a drug combination of an FAK inhibitor and a CD44 inhibitor for the treatment of cancer. The CD44 inhibitor and FAK inhibitor drug combination can have synergistic effects, significantly improving tumor inhibition effects, reducing toxic side effects, and overcoming tumor drug resistance, thereby providing a good choice for clinical treatment of cancer.



Processed by Luminess, 75001 PARIS (FR)

## Description

### Technical field

The present invention belongs to the field of medicine, and specifically relates to a pharmaceutical composition for treating cancers and its use.

### Background technology

Cancer usually refers to all malignant tumors, with biological characteristics such as abnormal cell differentiation and proliferation, uncontrolled growth, infiltration, and metastasis. Every year, there are many deaths due to various types of cancers, posing a serious threat to human survival. At present, the clinical treatment of tumors is still mainly based on surgery and chemotherapeutical drugs. However, since entering the 21st century, molecular targeted therapy is playing an increasingly important role. Compared to traditional cytotoxic medicaments, molecular targeted drugs have become a hot topic in the research and development of anti-tumor medicaments, since they target the characteristics of tumor cells that are different from normal cells, change blind attack to shooting the target, and thus reduce toxic side effects on normal organs and tissues, but play strong anti-tumor effects, thereby improving the life quality of patients.

CD44 is a transmembrane glycoprotein of cell surface, mainly involved in heterogeneous adhesion, which refers to the adhesion of tumor cells to host cells and host matrix. Heterogeneous adhesion plays a promoting role in the invasion and metastasis of tumor cells. The literature "Anti-CD44 antibodies inhibit both mTORC1 and mTORC2: a new rationale supporting CD44-induced AML differentiation therapy" (Merzaban, J, S, et al. Leukemia: Official journal of the Leukemia Society of America, Leukemia Research Fund, U.K, 2016) has indicated that inhibiting CD44 with antibodies can lead to a decrease in the expression of two main pathways involved in abnormal growth of cancer cells: the PI3K (phosphatidylinositol 3-kinase) pathway and the mTOR (mammalian rapamycin target protein) pathway. Therefore, CD44 inhibitors have the potential to be applied in the treatment of cancer. Although CD44 inhibitors exhibit good anticancer activity, they have been found to have high toxic side effects in clinical trials (References "CD44V6-targeted imaging of head and neck squamous cell carcinoma:antivody-based approaches" and "Hyaluronic acid targeting of CD44 for cancer therapy: from receptor biology to nanomedicine").

Focal adhesion kinase (FAK) is a non-receptor tyrosine protein kinase, which is an important cytoskeletal protein and key molecule in various signaling pathways in cells. FAK plays an important role in various stages of tumor occurrence, development, migration, and invasion. At present, FAK is regarded as a potential target for tumor treatment, and FAK inhibitors serve as ligands that can competitively bind to the binding site of FAK receptors with ATP, blocking the transmission of FAK-mediated growth and proliferation signaling pathways, leading to inhibition of malignant tumor cell growth and proliferation, and even leading to cell death at high doses. However, FAK inhibitors also have high toxic side effects. Moreover, with further research, it has been found that the biological role of FAK in the development of cancer conditions is complex, and its therapeutic effect on cancer cannot be determined.

In addition, drug resistance has always been a major challenge for the treatment of cancer. How to overcome the drug resistance is also a challenge in the study on cancer treatment. At present, there is no evidence of manufacturing a medicament for treating cancer by combining CD44 inhibitors and FAK inhibitors. Thereby, further research is needed to determine whether they have therapeutic effects on cancer, whether their toxic side effects are reduced, and whether they can overcome the drug resistance of cancer cells.

### Summary of the invention

The present invention is to provide a pharmaceutical composition for treating cancer and its use.

The present invention provided a pharmaceutical composition for treating cancer, which is composed of FAK inhibitors and CD44 inhibitors.

Further, the weight ratio of FAK inhibitor and CD44 inhibitor is 1:10-10:1.

Further, the FAK inhibitors are the following compounds, or optical isomers thereof, or tautomers thereof, or salts thereof, or prodrugs thereof, or hydrates thereof, or solvates thereof, and the following compounds include: Defactinib, CEP-28122, CEP-37440, TAE226, PF-562271, PF-431396, VS-4718, PF-573228, BI853520, IN10018, and APG-2449;

alternatively, the FAK inhibitor is a compound represented by formula I, or optical isomers thereof, or tautomers thereof, or salts thereof, or prodrugs thereof, or hydrates thereof, or solvates thereof,

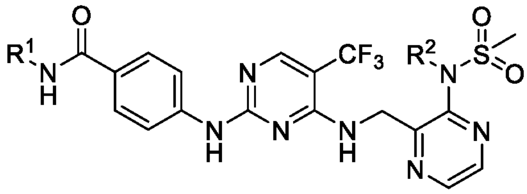

Formula I

wherein, $R^1$ and $R^2$ are each independently selected from the group consisting of hydrogen, methyl, and trideuteromethyl;

alternatively, the FAK inhibitor is the following compounds, or optical isomers thereof, or tautomers thereof, or salts thereof, or prodrugs thereof, or hydrates thereof, or solvates thereof, and the following compounds are selected from the group consisting of:

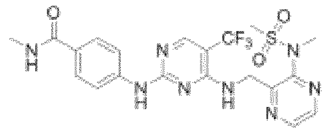
1

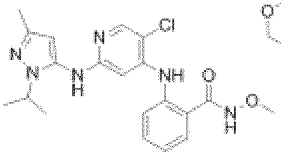
2

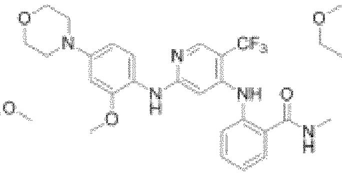
3

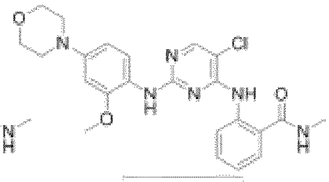
4

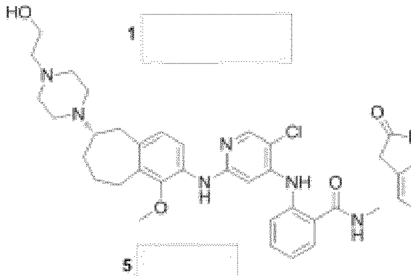
5

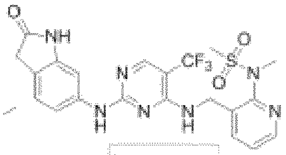
6

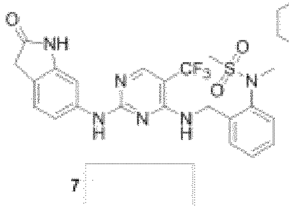
7

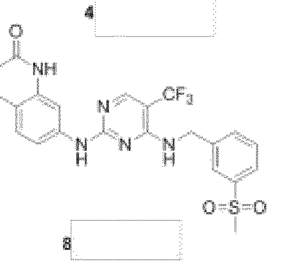
8

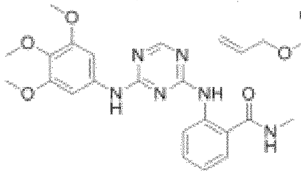
9

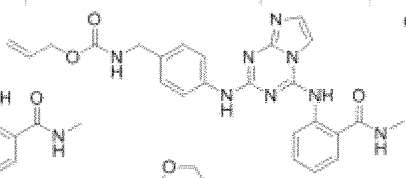
10

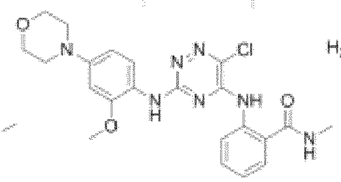
11

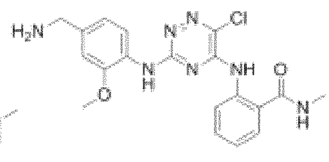
12

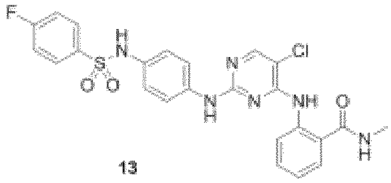
13

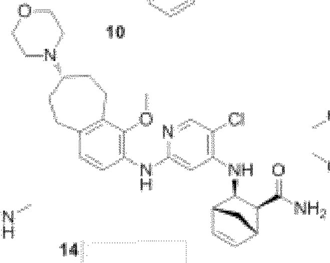
14

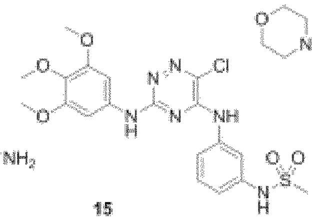
15

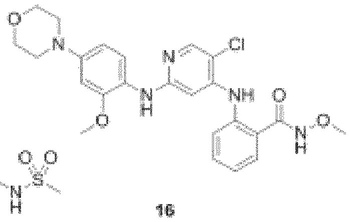
16

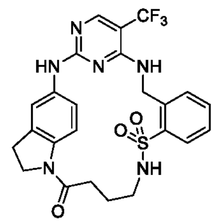
17

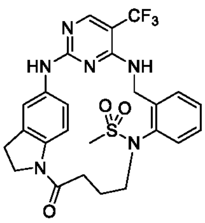
18

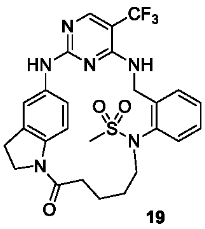
19

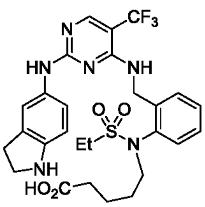
20

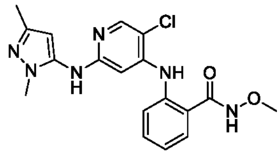
21

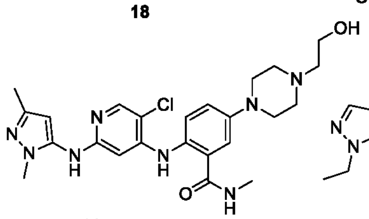
22

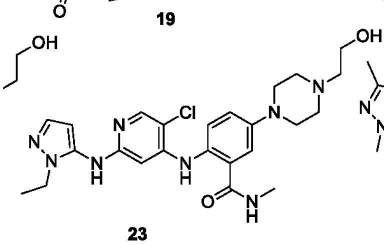
23

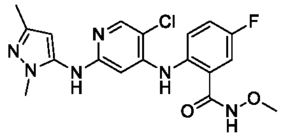
24

;

preferably, the FAK inhibitor is the following compounds, or optical isomers thereof, or tautomers thereof, or salts thereof, or prodrugs thereof, or hydrates thereof, or solvates thereof, and the following compounds include

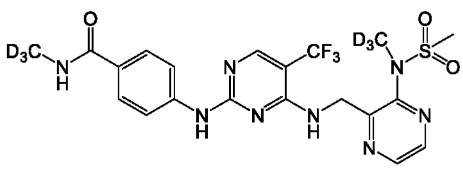
,

Defactinib or IN10018.

Further, the CD44 inhibitor is the following compounds, or optical isomers thereof, or tautomers thereof, or salts

thereof, or prodrugs thereof, or hydrates thereof, or solvates thereof, or peptides, or antibodies, and the following compounds or peptides or antibodies include Angstrom6, PEP-1, AMC303, Anti-CD44 mAb H4C4, anti-CD44 mAb HI44a, anti-CD44 mAb IM7, anti-CD44 mAb KM201, RO5429083, RG7356, anti-CD44V6 mAb 2F10, anti-CD44V6 mAb U36, anti-CD44V6 mAb V6B3, anti-CD44V6 mAb VFF18, anti-CD44V6 mAb VFF4, anti-CD44V6, and mAb VFF7;
preferably, the CD44 inhibitor is the following compound, or optical isomers thereof, or tautomers thereof, or salts thereof, or prodrugs thereof, or hydrates thereof, or solvates thereof, and the following compound includes AMC303.

Further, the molar ratio of FAK inhibitor and CD44 inhibitor is 1:82-82:1;
preferably, the molar ratio of FAK inhibitor and CD44 inhibitor is 0.4-81:1.

Further, the FAK inhibitor is compound

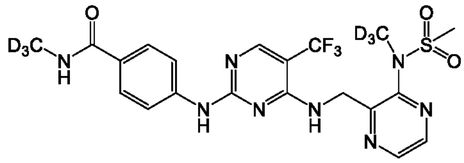,

or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 0.4-81:1;

alternatively, the FAK inhibitor is Defactinib, or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 0.4-27:1;
alternatively, the FAK inhibitor is IN 10018, or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 1:1.

The present invention also provides a preparation method for a pharmaceutical composition mentioned above, which comprises the following steps: FAK inhibitors and CD44 inhibitors were weighed according to the pre-determined weight ratio, and then well mixed.

The present invention also provides the use of the above pharmaceutical composition in the manufacturer of medicaments for treating cancer;

preferably, the cancer is solid tumor, mesothelioma, melanoma, prostate cancer, breast cancer, glioblastoma, and brain cancer;
the solid tumors include mesothelioma, pancreatic cancer, soft tissue tumor, metastatic tumor, non-solid cancer, sarcoma, adenocarcinoma, lung cancer, breast cancer, lymphoma, gastrointestinal cancer, urogenital system cancer, prostate cancer, ovarian cancer; the gastrointestinal cancer includes colon cancer, and the urogenital system cancer includes renal, urothelial, or testicular tumors; the ovarian cancer includes advanced ovarian cancer;
the mesothelioma includes neurofibroma, renal cancer, lung cancer, small cell lung cancer, non-small cell lung cancer, KRAS-mutant non-small cell lung cancer, liver cancer, thyroid cancer, breast cancer, nervous system tumor, neurilemmoma, meningioma, neuroma, adenoid cystic carcinoma, ependymoma, ependymal tumor, malignant pleural tumor, malignant pleural mesothelioma, triplet tumor, negative breast cancer, non-blood malignant tumor, melanoma, colorectal cancer, leukemia, adenocarcinoma, and solid tumors;
the melanoma includes locally advanced melanoma, locally mutant N-Ras driven melanoma, and metastatic malignant skin melanoma; the colorectal cancer includes metastatic colorectal cancer; the leukemia includes acute myelogenous leukemia; the adenocarcinoma includes adenocarcinoma; the solid tumors include locally advanced solid tumors, metastatic solid tumors, and hepatocellular carcinoma;
the prostate cancer includes castration-resistant prostate cancer and metastatic castration resistant prostate cancer;
the brain cancer includes neuroepithelial tissue tumors, cerebral gliomas, astrocytomas, oligodendrogliomas, ependymal and choroidal plexus tumors, pineal tumors, neurocellular tumors, gangliocytoma, neuroblastoma, poorly differentiated tumors, embryonal tumors, glioblastoma multiform, medulloblastoma, schwannoma, meningioma, malignant lymphoma, cerebrovascular tumors, vascular malformations, capillary telangiectases, pituitary tumors, and metastatic tumors.

The present invention also provides a pharmaceutical preparation for treating cancers, which is made from the above pharmaceutical compositions, as active ingredients, in combination with pharmaceutically acceptable excipients or auxiliary ingredients.

The present invention also provides a drug combination for treating cancers, which comprises FAK inhibitors and CD44 inhibitors in the same or different specifications administered simultaneously or separately, as well as pharmaceutically acceptable carriers;

preferably, the weight ratio of FAK inhibitor and CD44 inhibitor is 1:10-10:1;
alternatively, the molar ratio of FAK inhibitor and CD44 inhibitor is 1:82-82:1;
more preferably, the molar ratio of FAK inhibitor and CD44 inhibitor is 0.4-81:1.

Further, the FAK inhibitors are the following compounds, or optical isomers thereof, or tautomers thereof, or salts thereof, or prodrugs thereof, or hydrates thereof, or solvates thereof, and the following compounds include: Defactinib, CEP-28122, CEP-37440, TAE226, PF-562271, PF-431396, VS-4718, PF-573228, BI853520, IN10018, and APG-2449;

alternatively, the FAK inhibitor is a compound represented by formula I, or optical isomers thereof, or tautomers thereof, or salts thereof, or prodrugs thereof, or hydrates thereof, or solvates thereof,

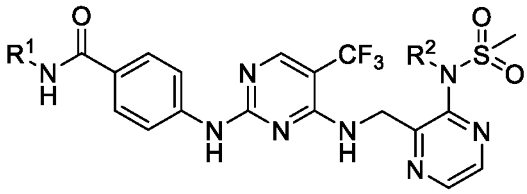

Formula I

wherein, $R^1$ and $R^2$ are each independently selected from the group consisting of hydrogen, methyl, and trideuteromethyl;
alternatively, the FAK inhibitor is the following compounds, or optical isomers thereof, or tautomers thereof, or salts thereof, or prodrugs thereof, or hydrates thereof, or solvates thereof, and the following compounds are selected from the group consisting of:

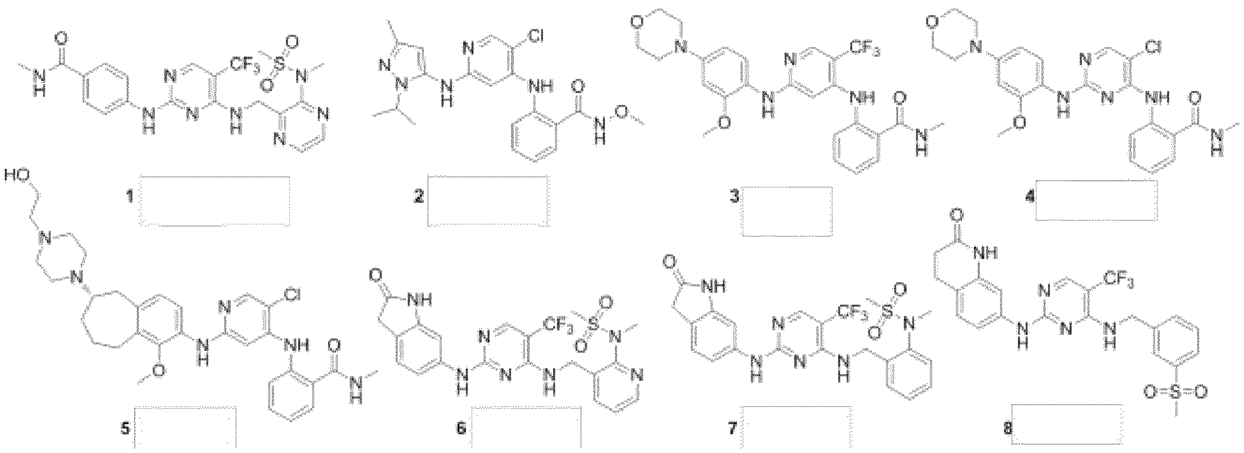

1 2 3 4

5 6 7 8

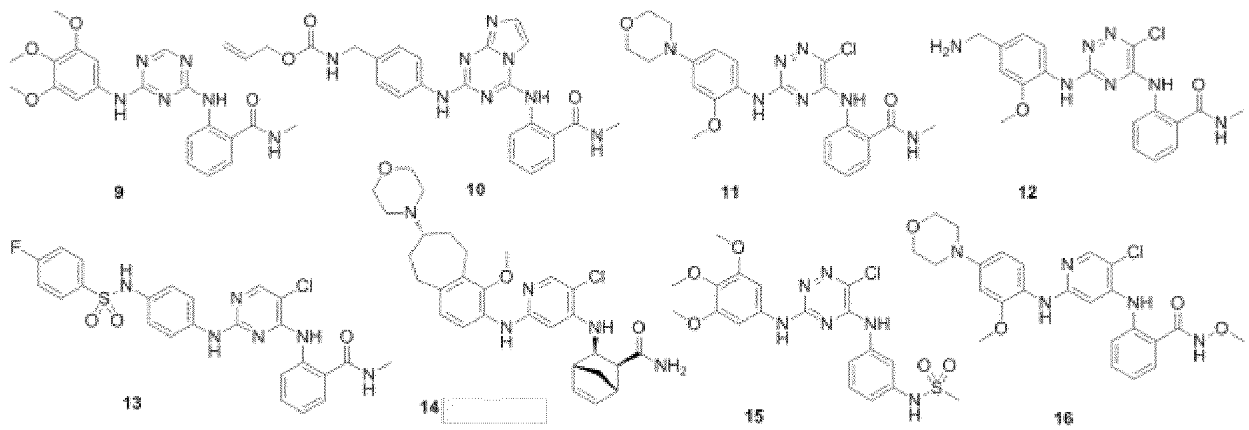

9 10 11 12

13 14 15 16

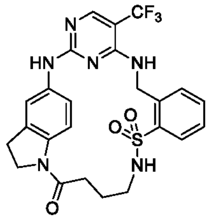

17

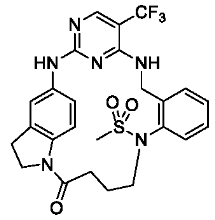

18

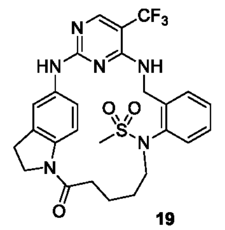

19

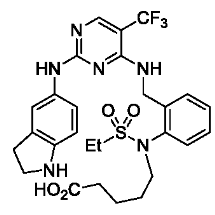

20

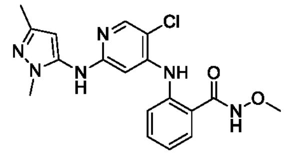

21

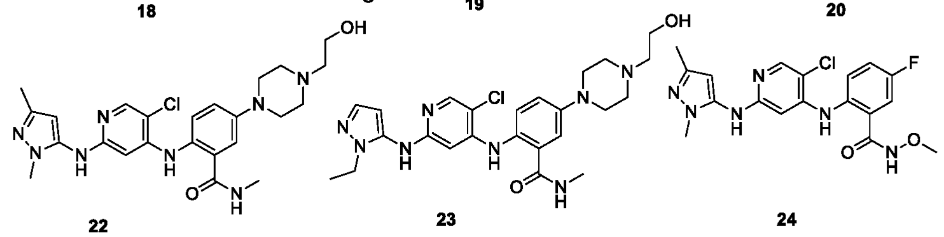

22 23 24

.

preferably, the FAK inhibitor is the following compounds, or optical isomers thereof, or tautomers thereof, or salts thereof, or prodrugs thereof, or hydrates thereof, or solvates thereof, and the following compounds include

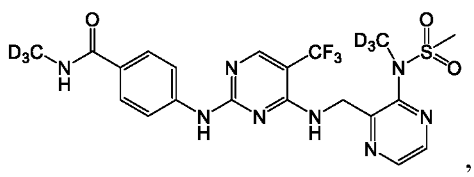

,

Defactinib or IN10018.

Further, the CD44 inhibitor is the following compounds, or optical isomers thereof, or tautomers thereof, or salts thereof, or prodrugs thereof, or hydrates thereof, or solvates thereof, or peptides, or antibodies, and the following compounds or peptides or antibodies include Angstrom6, PEP-1, AMC303, Anti-CD44 mAb H4C4, anti-CD44 mAb HI44a, anti-CD44 mAb IM7, anti-CD44 mAb KM201, RO5429083, RG7356, anti-CD44V6 mAb 2F10, anti-CD44V6 mAb U36, anti-CD44V6 mAb V6B3, anti-CD44V6 mAb VFF18, anti-CD44V6 mAb VFF4, anti-CD44V6 and mAb VFF7;
preferably, the CD44 inhibitor is the following compound, or optical isomers thereof, or tautomers thereof, or salts thereof, or prodrugs thereof, or hydrates thereof, or solvates thereof, and the following compound includes AMC303.

Further, the FAK inhibitor is compound

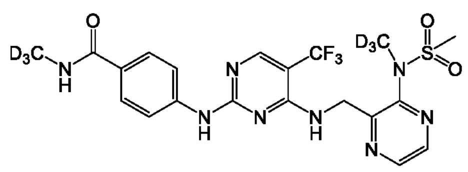

,

or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 0.4-81:1;

alternatively, the FAK inhibitor is Defactinib, or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 0.4-27:1;
alternatively, the FAK inhibitor is IN 10018, or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 1:1.

The present invention also provides the use of FAK inhibitors combined with CD44 inhibitors in the manufacturer of medicaments for treating cancer;

preferably, the weight ratio of FAK inhibitor and CD44 inhibitor is 1:10-10:1;
alternatively, the molar ratio of FAK inhibitor and CD44 inhibitor is 1:82-82:1;
more preferably, the molar ratio of FAK inhibitor and CD44 inhibitor is 0.4-81:1.

Further, the FAK inhibitors are the following compounds, or optical isomers thereof, or tautomers thereof, or salts thereof, or prodrugs thereof, or hydrates thereof, or solvates thereof, and the following compounds include: Defactinib, CEP-28122, CEP-37440, TAE226, PF-562271, PF-431396, VS-4718, PF-573228, BI853520, IN10018, and APG-2449;

alternatively, the FAK inhibitor is a compound represented by formula I, or optical isomers thereof, or tautomers thereof, or salts thereof, or prodrugs thereof, or hydrates thereof, or solvates thereof,

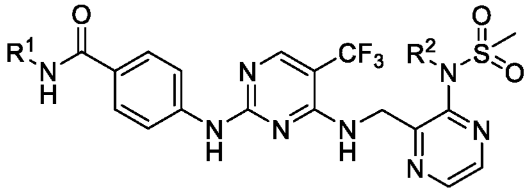

Formula I

wherein, $R^1$ and $R^2$ are each independently selected from the group consisting of hydrogen, methyl, and trideuteromethyl;
alternatively, the FAK inhibitor is the following compounds, or optical isomers thereof, or tautomers thereof, or salts thereof, or prodrugs thereof, or hydrates thereof, or solvates thereof, and the following compounds are selected from the group consisting of:

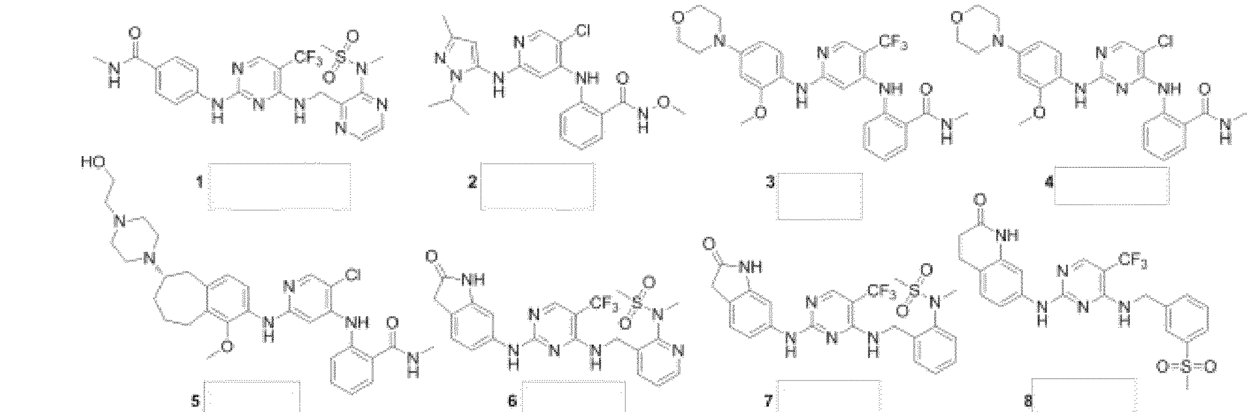

1 2 3 4

5 6 7 8

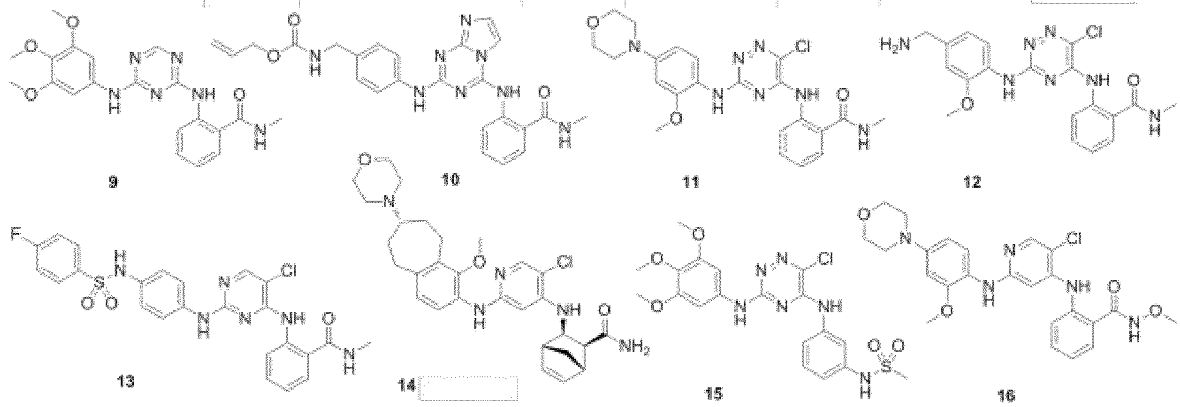

9 10 11 12

13 14 15 16

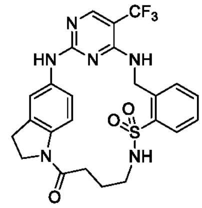

17

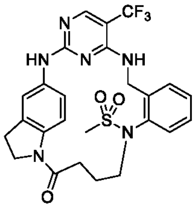

18

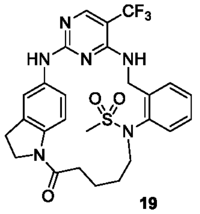

19

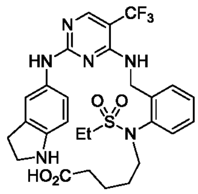

20

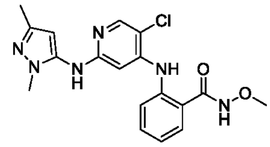

21

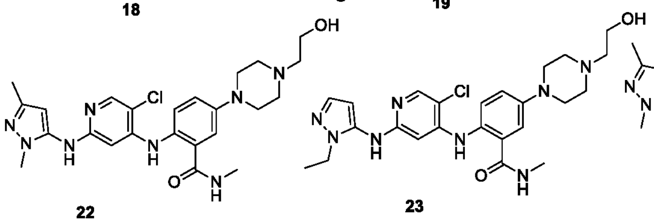

22 23

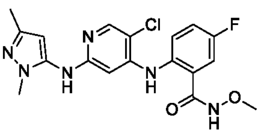

24

;

preferably, the FAK inhibitor is the following compounds, or optical isomers thereof, or tautomers thereof, or salts thereof, or prodrugs thereof, or hydrates thereof, or solvates thereof, and the following compounds include

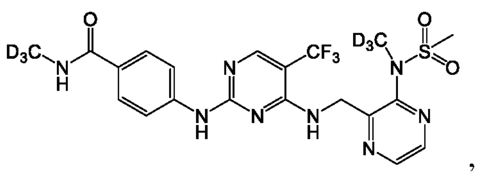

,

Defactinib or IN10018.

Further, the CD44 inhibitor is the following compounds, or optical isomers thereof, or tautomers thereof, or salts thereof, or prodrugs thereof, or hydrates thereof, or solvates thereof, or peptides, or antibodies, and the following compounds or peptides or antibodies include Angstrom6, PEP-1, AMC303, Anti-CD44 mAb H4C4, anti-CD44 mAb HI44a,

anti-CD44 mAb IM7, anti-CD44 mAb KM201, RO5429083, RG7356, anti-CD44V6 mAb 2F10, anti-CD44V6 mAb U36, anti-CD44V6 mAb V6B3, anti-CD44V6 mAb VFF18, anti-CD44V6 mAb VFF4, anti-CD44V6, and mAb VFF7; preferably, the CD44 inhibitor is the following compound, or optical isomers thereof, or tautomers thereof, or salts thereof, or prodrugs thereof, or hydrates thereof, or solvates thereof, and the following compound includes AMC303.

Further, the FAK inhibitor is compound

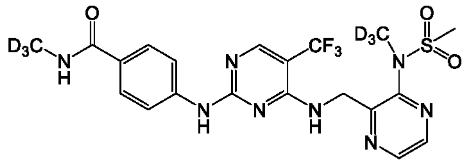,

or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 0.4-81:1;

alternatively, the FAK inhibitor is Defactinib, or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 0.4-27:1;
alternatively, the FAK inhibitor is IN 10018, or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 1:1.

Further, the cancer is solid tumor, mesothelioma, melanoma, prostate cancer, breast cancer, glioblastoma, and brain cancer;

the solid tumors include mesothelioma, pancreatic cancer, soft tissue tumor, metastatic tumor, non-solid cancer, sarcoma, adenocarcinoma, lung cancer, breast cancer, lymphoma, gastrointestinal cancer, urogenital system cancer, prostate cancer, ovarian cancer; the gastrointestinal cancer includes colon cancer, and the urogenital system cancer includes renal, urothelial or testicular tumors; the ovarian cancer includes advanced ovarian cancer;
the mesothelioma includes neurofibroma, renal cancer, lung cancer, small cell lung cancer, non-small cell lung cancer, KRAS-mutant non-small cell lung cancer, liver cancer, thyroid cancer, breast cancer, nervous system tumor, neurilemmoma, meningioma, neuroma, adenoid cystic carcinoma, ependymoma, ependymal tumor, malignant pleural tumor, malignant pleural mesothelioma, triplet tumor, negative breast cancer, non-blood malignant tumor, melanoma, colorectal cancer, leukemia, adenocarcinoma, and solid tumors;
the melanoma includes locally advanced melanoma, locally mutant N-Ras driven melanoma, and metastatic malignant skin melanoma; the colorectal cancer includes metastatic colorectal cancer; the leukemia includes acute myelogenous leukemia; the adenocarcinoma includes adenocarcinoma; the solid tumors include locally advanced solid tumors, metastatic solid tumors, and hepatocellular carcinoma;
the prostate cancer includes castration-resistant prostate cancer and metastatic castration resistant prostate cancer; the brain cancer includes neuroepithelial tissue tumors, cerebral gliomas, astrocytomas, oligodendrogliomas, ependymal and choroidal plexus tumors, pineal tumors, neurocellular tumors, gangliocytoma, neuroblastoma, poorly differentiated tumors, embryonal tumors, glioblastoma multiform, medulloblastoma, schwannoma, meningioma, malignant lymphoma, cerebrovascular tumors, vascular malformations capillary telangiectases, pituitary tumors, and metastatic tumors.

The drug combination of CD44 inhibitors and FAK inhibitors according to the present invention can play a synergistic effect, significantly improve the inhibitory effect on tumors, reduce toxic side effects, and overcome tumor drug resistance, thereby providing a good choice for clinical treatment of cancer.

Obviously, based on the above content of the present invention, according to the common technical knowledge and the general means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the

present invention.

**Description of Figures**

Figure 1. Histogram of survival rate for compound **25** combined with AMC303 against A549 cells.
Figure 2. Histogram of survival rate for Defactinib combined with AMC303 against A549 cells.
Figure 3. Images of wound healing assay for compound **25,** Defactinib or IN10018 combined with AMC303 against A549 cells.
Figure 4. Statistical chart of wound healing assay for compound **25,** Defactinib or IN10018 combined with AMC303 against A549 cells.
Figure 5. Histogram of survival rate for compound **25** combined with AMC303 against SW620 cells.
Figure 6. Histogram of survival rate for Defactinib combined with AMC303 against SW620 cells.

**Examples**

The starting materials and equipment used in the specific examples of the present invention are known products obtained by purchasing those commercially available.

In the examples of the present invention, the structure of compound **25** is

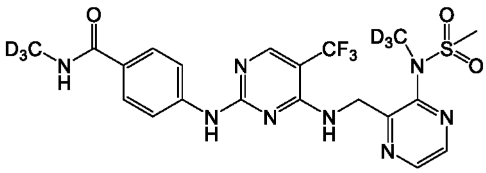

,

which was prepared according to the method disclosed in the patent application number 201910373081.2. Defactinib is a commercially available FAK inhibitor. AMC303 is a commercially available CD44v6 inhibitor developed by amcure GmbH. IN 10018 is a commercially available FAK inhibitor.

**Example 1: The inhibitory effect of the pharmaceutical composition according to the present invention on the proliferation of cancer cells**

**1. Experimental methods**

Non-small cell lung cancer A549 cells in the logarithmic growth phase were seeded in a 96-well plate at a concentration of $1.0 \times 10^3$ cells/well. The plate was placed in an incubator and cultured at 37 °C under 5% $CO_2$ and saturated humidity for 24 h. After 24 hours of incubation, the drugs FAK inhibitor (compound **25** or Defactinib) and CD44 inhibitor (AMC303) were added successively to the 96-well plate. RPMI 1640 cell culture medium containing 10% fetal bovine serum was added to the negative control wells in a volume same as that of the drug.

The concentration of compound **25** was: 0 μM, 0.123 μM, 0.370 μM, 1.111 μM, 3.333 μM, and 10 μM
The concentration of Defactinib was: 0 μM, 0.123 μM, 0.370 μM, 1.111 μM, 3.333 μM, and 10 μM
The concentration of AMC303 was: 0 μM, 0.002 μM, 0.005 μM, 0.014 μM, 0.041 μM, 0.123 μM, 0.370 μM, 1.111 μM, 3.333 μM, and 10 μM.

After co-incubation with drugs for 6 days, 10 μL of CCK-8 was added to each well, followed by cultivating for additional 2.5 h. The absorbance value (OD) of each well was measured using an enzyme-linked immunosorbent assay (ELISA) at a wavelength of 450 nm. The inhibition rate of cell growth was calculated according to the formula, that is, Inhibition rate (%) = (1- OD value of test well/OD value of control well) × 100%. Then, the effect was determined based on the drug combination index q=Ea+b/(Ea+Eb-Ea*Eb). The judgment criteria for q value is shown in Table 1:

Table 1. Judgment criteria for q value.

| q | Description |
|---|---|
| > 1 | Synergistic effect |

(continued)

| q | Description |
|---|---|
| < 1 | Antagonistic effect |

## 2. Experimental results

### (1) Combination of compound 25 and AMC303 (compound 25 & AMC303)

The results for the combination of compound **25** and AMC303 are shown in Figure 1. In Figure 1, when the molar ratio of compound **25** and AMC303 was 81:1, the combination of compound **25** and AMC303 had a synergistic effect on inhibiting the growth of A549 cells, and thereby could be used for synergistic treatment of non-small cell lung cancer.

### (2) Combination of Defactinib and AMC303 (Defactinib&AMC303)

The results for the combination of Defactinib and AMC303 are shown in Figure 2. As shown in Figure 2, when the molar ratio of Defactinib and AMC303 was 27:1, the drug combination of Defactinib and AMC303 had a synergistic effect on inhibiting the growth of A549 cells, and thereby could be used for synergistically treating non-small cell lung cancer.

The above example provided a drug combination for the treatment of non-small cell lung cancer. The experimental results indicated that the combination of FAK inhibitor (compound **25** or Defactinib) and CD44 inhibitor AMC303 could significantly inhibit the growth and proliferation of non-small cell lung cancer cells, and the anticancer effect of the combination was significantly better than that of FAK inhibitor (compound **25** or Defactinib) and CD44 inhibitor AMC303 used alone, suggesting a synergistic effect. The drug combination of the present invention has good clinical application prospects in the treatment of non-small cell lung cancer.

## Example 2: The effect of the pharmaceutical composition according to the present invention on inhibiting the migration of cancer cells

## 1. Experimental method

Non-small cell lung cancer A549 cells in logarithmic growth phase were collected, and then seeded into a 12-well plate at a concentration of $1.0 \times 10^6$ cells/well. The plate was placed in an incubator at 37 °C under 5% $CO_2$ and saturated humidity, and then cultured for 24 hours. The cells in the 12-well plate were assigned with 3 scratches per well. After scratching, the media were discarded, and each well was further rinsed with PBS to remove floating cells. FAK inhibitors (compound **25,** Defactinib, or IN10018) and CD44 inhibitors (AMC303) were successively added to the 12-well plate. The drugs were dissolved in serum-free RPMI 1640 cell culture medium containing 20 ng/ml HGF. Serum-free RPMI 1640 cell culture medium (containing 20 ng/ml HGF) was also added to the negative control well in the same volume as that of the drug solution.

Concentration of compound **25:** 0.4 μM;
Concentration of Defactinib: 0.4 μM;
Concentration of IN 100 18: 1 μM;
Concentration of AMC303: 1 μM.

After adding 4 drugs separately (compound **25,** Defactinib, IN10018, AMC303), or simultaneously adding compound **25** and AMC303 (compound **25**&AMC303, at a concentration of 0.4 μM for compound **25** and 1 μM for AMC303), or simultaneously adding Defactinib and AMC303 (Defactinib&AMC303, at a concentration of 0.4 μM for Defactinib and 1 μM for AMC303), or simultaneously adding IN10018 and AMC303 (IN10018&AMC303, at a concentration of 1 μM for IN10018 and 1 μM for AMC303), the plate was incubated for 24 hours, and then the culture medium was discarded. The plate was rinsed with PBS to remove floating cells. The migration of cells was observed under a microscope, and the image was produced by the action of eyepiece 10× and objective 10× (images were taken at 0 h and 24 h, respectively). Image processing was performed using Image J, to analyze the migration rate of cells. The formula for calculating the migration rate:

$$\text{Migration rate (\%)} = [\text{Scratch area (0 h)-Scratch area (48 h)}]/\ \text{Scratch area (0 h)} \times 100\%$$

Based on the migration rate, a histogram was created by Prism Statistics, and then T-test was carried out. $p < 0.05$ means a significant difference.

**2. Experimental results**

The results of cell migration are shown in Figures 3-4 and Table 2.

Table 2. The statistics of significant differences in cell migration results among groups.

| Pharmaceutical compositions | P values |
|---|---|
| Compound **25**&AMC303 vs Compound **25** | 0.046 |
| Compound **25**&AMC303 vs AMC303 | 0.036 |
| Defactinib&AMC303 vs Defactinib | 0.028 |
| Defactinib&AMC303 vs AMC303 | 0.016 |
| IN10018&AMC303 vs IN10018 | 0.045 |
| IN10018&AMC303 vs AMC303 | 0.014 |

As shown in Figures 3-4 and Table 2, the combination of FAK inhibitors (compound **25,** Defactinib, or IN10018) and CD44 inhibitor AMC303 could significantly inhibit the migration of non-small cell lung cancer cells, and the anticancer effect of the combination was significantly better than that of FAK inhibitors (compound **25,** Defactinib, or IN10018) and CD44 inhibitor AMC303 alone, indicating a synergistic effect. The drug combination of the present invention had good clinical application prospects in the treatment of non-small cell lung cancer.

**Example 3: The inhibitory effect of the pharmaceutical composition according to the present invention on the proliferation of cancer cells**

**1. Experimental methods**

Colorectal cancer SW620 cells in logarithmic growth phase were seeded in a 96-well plate at a concentration of $1.0 \times 10^3$ cells/well. The plate was cultured in an incubator at 37 °C under 5% $CO_2$ and saturated humidity for 24 h. After 24 hours of incubation, the drugs FAK inhibitor (compound **25** or Defactinib) and CD44 inhibitor (AMC303) were added successively to the 96-well plate. RPMI 1640 cell culture medium containing 10% fetal bovine serum was added to the negative control wells in a volume same as that of the drug.

The concentration of compound **25** was: 0 μM, 0.123 μM, 0.370 μM, 1.111 μM, 3.333 μM, and 10 μM
The concentration of Defactinib was: 0 μM, 0.123 μM, 0.370 μM, 1.111 μM, 3.333 μM, and 10 μM
The concentration of AMC303 was: 0 μM, 0.002 μM, 0.005 μM, 0.014 μM, 0.041 μM, 0.123 μM, 0.370 μM, 1.111 μM, 3.333 μM, and 10 μM.

After co-incubation with drugs for 6 days, 10 μL of CCK-8 was added to each well, followed by culturing for additional 2.5 h. The absorbance value (OD) of each well was measured with ELISA at a wavelength of 450 nm. The inhibition rate of cell growth was calculated according to the formula:

$$\text{Inhibition rate (\%)} = (1\text{- OD value of test well/OD value of control well}) \times 100\%.$$

Then, the effect was determined based on the drug combination index q=Ea+b/(Ea+Eb-Ea*Eb). The judgment criteria for q value is shown in Table 3:

Table 3. Judgment criteria for q value.

| q | Description |
|---|---|
| > 1 | Synergistic effect |
| < 1 | Antagonistic effect |

**2. Experimental results**

**(1) Combination of compound 25 and AMC303 (compound 25&AMC303)**

The results for the combination of compound **25** and AMC303 are shown in Figure 5. Based on Figure 1, when the molar ratio of compound **25** and AMC303 was 81:1, the combination of compound **25** and AMC303 had a synergistic effect on inhibiting the growth of SW620 cells, and thus could be used for synergistically treating colorectal cancer.

**(2) Combination of Defactinib and AMC303 (Defactinib&AMC303)**

The results for the combination of Defactinib and AMC303 are shown in Figure 6. As shown in Figure 6, when the molar ratio of Defactinib and AMC303 was 1:1, the combination of Defactinib and AMC303 had a synergistic effect on inhibiting the growth of SW620 cells, and so could be used for synergistically treating colorectal cancer.

The above example provided a drug combination for the treatment of colorectal cancer. The experimental results indicated that the combination of FAK inhibitor (compound **25** or Defactinib) and CD44 inhibitor AMC303 could significantly inhibit the growth and proliferation of colorectal cells, and the anticancer effect of the combination was significantly better than that of FAK inhibitor (compound **25** or Defactinib) and CD44 inhibitor AMC303 alone, suggesting a synergistic effect. The drug combination of the present invention had good clinical application prospects in the treatment of colorectal cancer.

In summary, the inventors had found that the combination of CD44 inhibitor and FAK inhibitor was demonstrated to have a synergistic effect, significantly improved the inhibitory effect on tumors, and overcame tumor drug resistance, thereby providing a good choice for clinical treatment of cancer.

**Claims**

1. A pharmaceutical composition for treating cancers, **characterized in that** it is composed of FAK inhibitors and CD44 inhibitors.

2. The pharmaceutical composition according to claim 1, **characterized in that** the weight ratio of FAK inhibitor and CD44 inhibitor is 1:10-10:1.

3. The pharmaceutical composition according to claim 1 or 2, **characterized in that** the FAK inhibitors are the following compounds, or optical isomers thereof, or tautomers thereof, or salts thereof, or prodrugs thereof, or hydrates thereof, or solvates thereof, and the following compounds include: Defactinib, CEP-28122, CEP-37440, TAE226, PF-562271, PF-431396, VS-4718, PF-573228, BI853520, IN10018, and APG-2449;

   alternatively, the FAK inhibitor is a compound represented by formula I, or optical isomers thereof, or tautomers thereof, or salts thereof, or prodrugs thereof, or hydrates thereof, or solvates thereof,

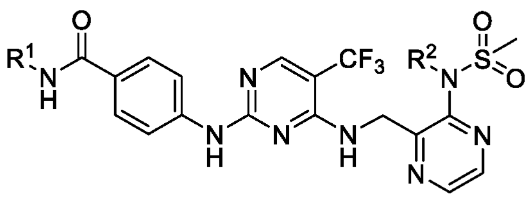

Formula I

   wherein, $R^1$ and $R^2$ are each independently selected from the group consisting of hydrogen, methyl, and trideuteromethyl;

alternatively, the FAK inhibitor is the following compounds, or optical isomers thereof, or tautomers thereof, or salts thereof, or prodrugs thereof, or hydrates thereof, or solvates thereof, and the following compounds are selected from the group consisting of:

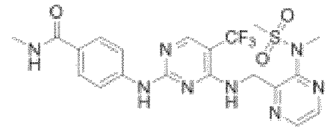
1

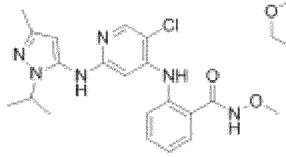
2

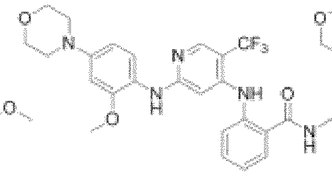
3

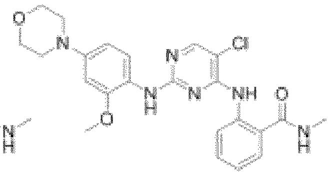
4

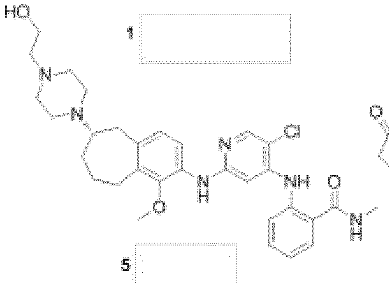
5

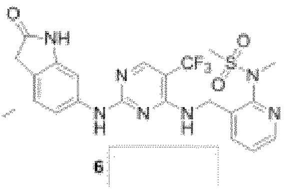
6

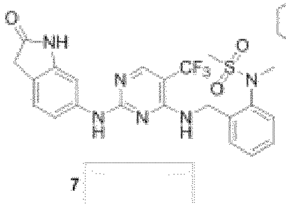
7

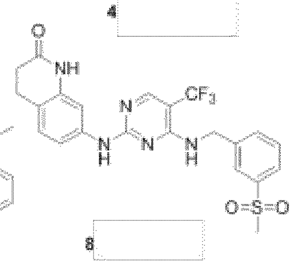
8

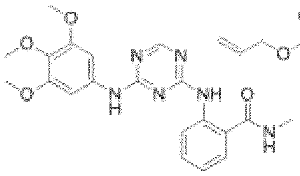
9

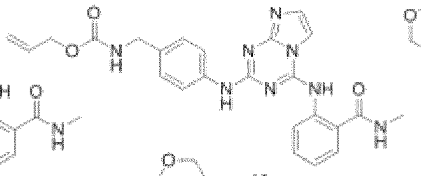
10

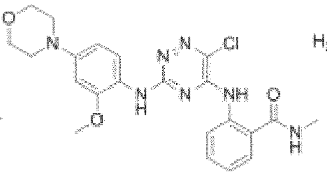
11

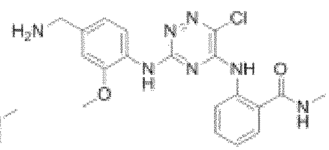
12

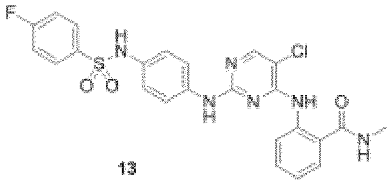
13

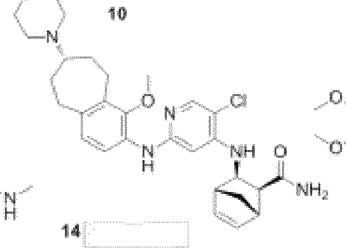
14

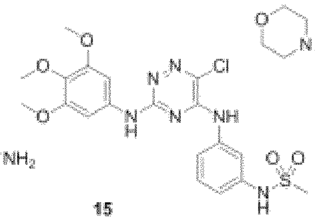
15

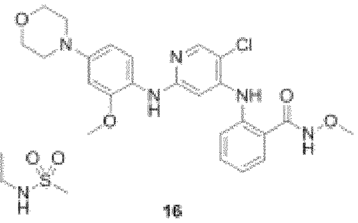
16

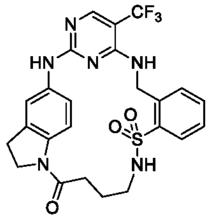
17

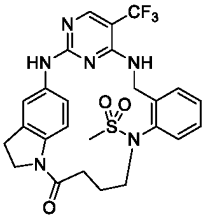
18

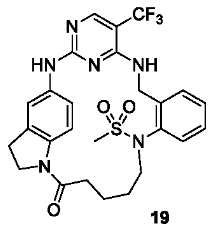
19

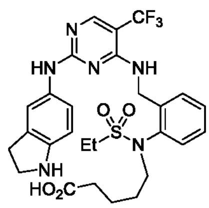
20

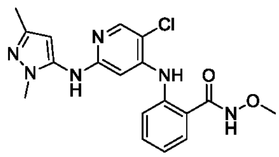
21

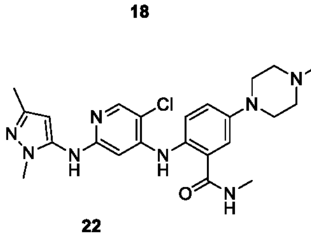
22

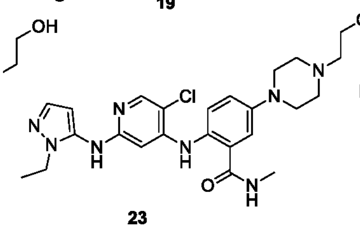
23

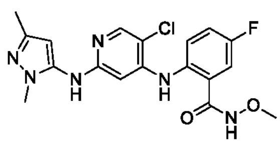
24

;

preferably, the FAK inhibitor is the following compounds, or optical isomers thereof, or tautomers thereof, or salts thereof, or prodrugs thereof, or hydrates thereof, or solvates thereof, and the following compounds include

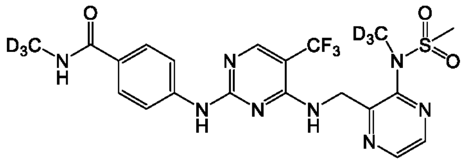

,

Defactinib or IN10018.

4. The pharmaceutical composition according to claim 1 or 2, **characterized in that** the CD44 inhibitor is the following compounds, or optical isomers thereof, or tautomers thereof, or salts thereof, or prodrugs thereof, or hydrates thereof, or solvates thereof, or peptides, or antibodies, and the following compounds or peptides or antibodies include Angstrom6, PEP-1, AMC303, Anti-CD44 mAb H4C4, anti-CD44 mAb HI44a, anti-CD44 mAb IM7, anti-CD44 mAb KM201, RO5429083, RG7356, anti-CD44V6 mAb 2F10, anti-CD44V6 mAb U36, anti-CD44V6 mAb V6B3, anti-CD44V6 mAb VFF18, anti-CD44V6 mAb VFF4, anti-CD44V6 and mAb VFF7;
preferably, the CD44 inhibitor is the following compound, or optical isomers thereof, or tautomers thereof, or salts thereof, or prodrugs thereof, or hydrates thereof, or solvates thereof, and the following compound includes AMC303.

5. The pharmaceutical composition according to claim 1 or 2, **characterized in that** the molar ratio of FAK inhibitor and CD44 inhibitor is 1:82-82:1;
preferably, the molar ratio of FAK inhibitor and CD44 inhibitor is 0.4-81:1.

6. The pharmaceutical composition according to claim 1 or 2, **characterized in that** the FAK inhibitor is compound

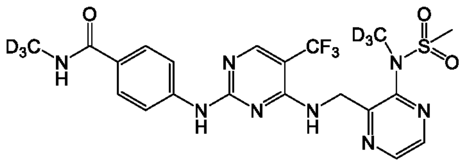

,

or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 0.4-81:1;

alternatively, the FAK inhibitor is Defactinib, or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 0.4-27:1;
alternatively, the FAK inhibitor is IN 10018, or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 1:1.

7. A preparation method for a pharmaceutical composition according to any one of claims 1 to 6, **characterized in that** it comprises the following steps: FAK inhibitors and CD44 inhibitors were weighed according to the pre-determined weight ratio, and then well mixed.

8. The pharmaceutical composition according to any one of claims 1 to 6 for use in the manufacturer of medicaments for treating cancers;

preferably, the cancer is solid tumor, mesothelioma, melanoma, prostate cancer, breast cancer, glioblastoma, and brain cancer;
the solid tumors include mesothelioma, pancreatic cancer, soft tissue tumor, metastatic tumor, non-solid cancer, sarcoma, adenocarcinoma, lung cancer, breast cancer, lymphoma, gastrointestinal cancer, urogenital system cancer, prostate cancer, ovarian cancer; the gastrointestinal cancer includes colon cancer, and the urogenital system cancer includes renal, urothelial, or testicular tumors; the ovarian cancer includes advanced ovarian cancer;

the mesothelioma includes neurofibroma, renal cancer, lung cancer, small cell lung cancer, non-small cell lung cancer, KRAS-mutant non-small cell lung cancer, liver cancer, thyroid cancer, breast cancer, nervous system tumor, neurilemmoma, meningioma, neuroma, adenoid cystic carcinoma, ependymoma, ependymal tumor, malignant pleural tumor, malignant pleural mesothelioma, triplet tumor, negative breast cancer, non-blood malignant tumor, melanoma, colorectal cancer, leukemia, adenocarcinoma, and solid tumors;

the melanoma includes locally advanced melanoma, locally mutant N-Ras driven melanoma, and metastatic malignant skin melanoma; the colorectal cancer includes metastatic colorectal cancer; the leukemia includes acute myelogenous leukemia; the adenocarcinoma includes adenocarcinoma; the solid tumors include locally advanced solid tumors, metastatic solid tumors, and hepatocellular carcinoma;

the prostate cancer includes castration-resistant prostate cancer and metastatic castration resistant prostate cancer;

the brain cancer includes neuroepithelial tissue tumors, cerebral gliomas, astrocytomas, oligodendrogliomas, ependymal and choroidal plexus tumors, pineal tumors, neurocellular tumors, gangliocytoma, neuroblastoma, poorly differentiated tumors, embryonal tumors, glioblastoma multiform, medulloblastoma, schwannoma, meningioma, malignant lymphoma, cerebrovascular tumors, vascular malformations, capillary telangiectases, pituitary tumors, and metastatic tumors.

**9.** A pharmaceutical preparation for treating cancers, **characterized in that** it is made from the pharmaceutical compositions according to any one of claims 1 to 6, as active ingredients, in combination with pharmaceutically acceptable excipients or auxiliary ingredients.

**10.** A drug combination for treating cancers, **characterized in that** it comprises FAK inhibitors and CD44 inhibitors in the same or different specifications administered simultaneously or separately, as well as pharmaceutically acceptable carriers;

preferably, the weight ratio of FAK inhibitor and CD44 inhibitor is 1:10-10:1;
alternatively, the molar ratio of FAK inhibitor and CD44 inhibitor is 1:82-82:1;
more preferably, the molar ratio of FAK inhibitor and CD44 inhibitor is 0.4-81:1.

**11.** The combination drug according to claim 10, **characterized in that** the FAK inhibitors are the following compounds, or optical isomers thereof, or tautomers thereof, or salts thereof, or prodrugs thereof, or hydrates thereof, or solvates thereof, and the following compounds include: Defactinib, CEP-28122, CEP-37440, TAE226, PF-562271, PF-431396, VS-4718, PF-573228, BI853520, IN10018, andAPG-2449;

alternatively, the FAK inhibitor is a compound represented by formula I, or optical isomers thereof, or tautomers thereof, or salts thereof, or prodrugs thereof, or hydrates thereof, or solvates thereof,

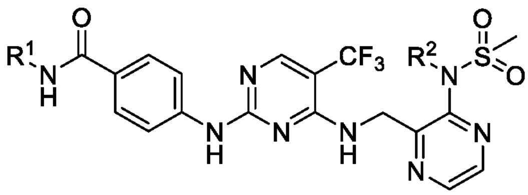

Formula I

wherein, $R^1$ and $R^2$ are each independently selected from the group consisting of hydrogen, methyl, and trideuteromethyl;

alternatively, the FAK inhibitor is the following compounds, or optical isomers thereof, or tautomers thereof, or salts thereof, or prodrugs thereof, or hydrates thereof, or solvates thereof, and the following compounds are selected from the group consisting of:

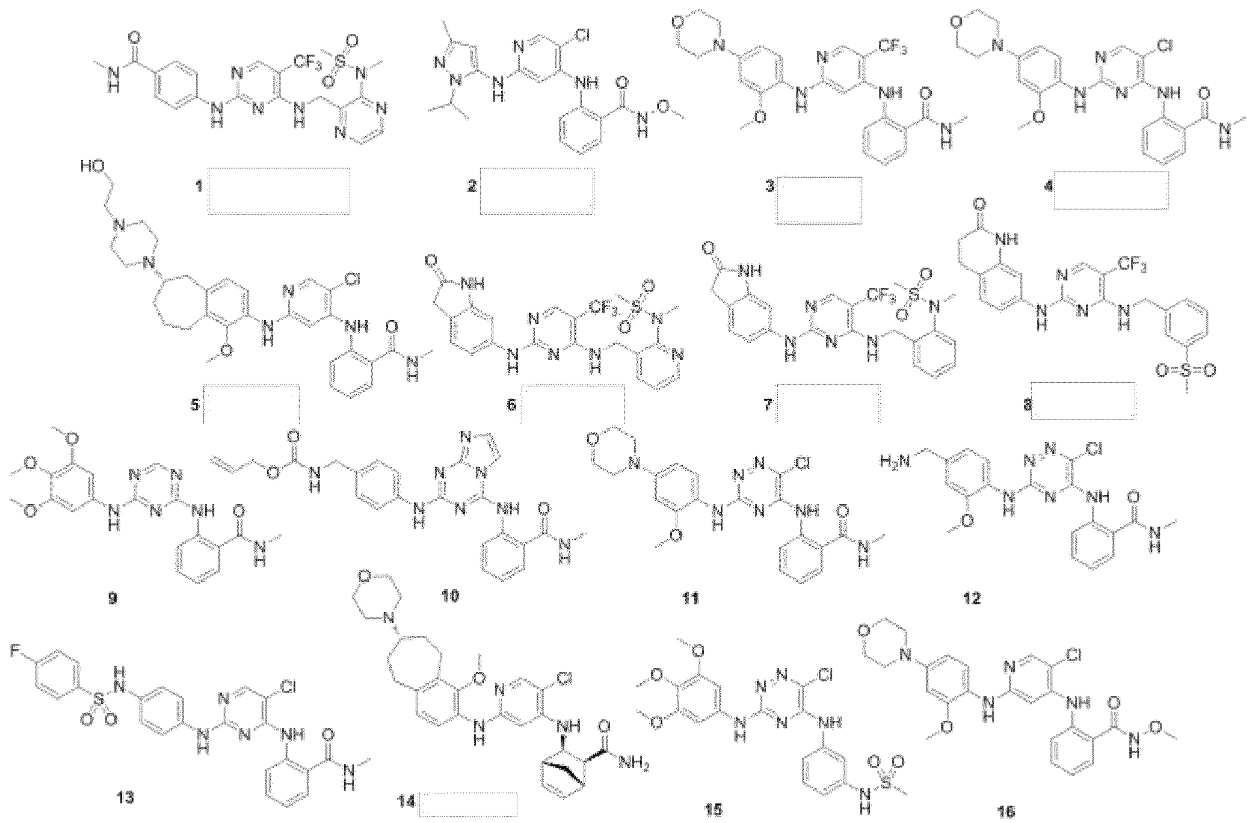

1 2 3 4

5 6 7 8

9 10 11 12

13 14 15 16

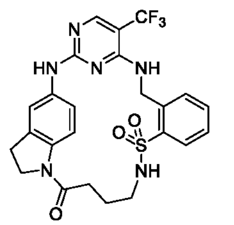

17

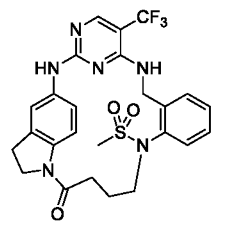

18

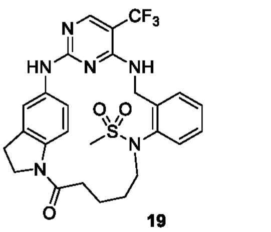

19

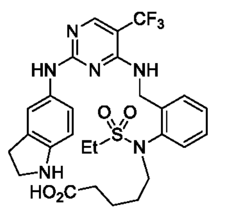

20

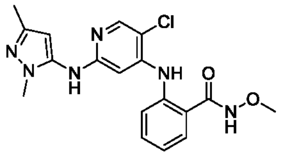

21

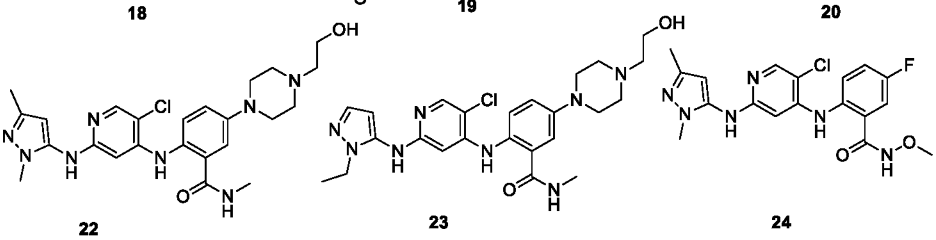

22 23 24

;

preferably, the FAK inhibitor is the following compounds, or optical isomers thereof, or tautomers thereof, or salts thereof, or prodrugs thereof, or hydrates thereof, or solvates thereof, and the following compounds include

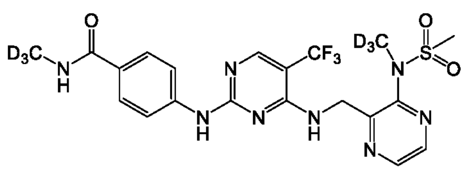

,

Defactinib or IN10018.

**12.** The drug combination according to claim 10, **characterized in that** the CD44 inhibitor is the following compounds, or optical isomers thereof, or tautomers thereof, or salts thereof, or prodrugs thereof, or hydrates thereof, or solvates thereof, or peptides, or antibodies, and the following compounds or peptides or antibodies include Angstrom6, PEP-1, AMC303, Anti-CD44 mAb H4C4, anti-CD44 mAb HI44a, anti-CD44 mAb IM7, anti-CD44 mAb KM201, RO5429083, RG7356, anti-CD44V6 mAb 2F10, anti-CD44V6 mAb U36, anti-CD44V6 mAb V6B3, anti-CD44V6 mAb VFF18, anti-CD44V6 mAb VFF4, anti-CD44V6 and mAb VFF7;
preferably, the CD44 inhibitor is the following compound, or optical isomers thereof, or tautomers thereof, or salts thereof, or prodrugs thereof, or hydrates thereof, or solvates thereof, and the following compound includes AMC303.

**13.** The drug composition according to any one of claims 10 to 12, **characterized in that** the FAK inhibitor is compound

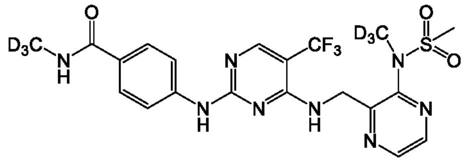,

or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 0.4-81:1;

alternatively, the FAK inhibitor is Defactinib, or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 0.4-27:1;
alternatively, the FAK inhibitor is IN 10018, or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 1:1.

**14.** The use of FAK inhibitors combined with CD44 inhibitors in the manufacturer of medicaments for treating cancers;

preferably, the weight ratio of FAK inhibitor and CD44 inhibitor is 1:10-10:1;
alternatively, the molar ratio of FAK inhibitor and CD44 inhibitor is 1:82-82:1;
more preferably, the molar ratio of FAK inhibitor and CD44 inhibitor is 0.4-81:1.

**15.** The use according to claim 14, **characterized in that** the FAK inhibitors are the following compounds, or optical isomers thereof, or tautomers thereof, or salts thereof, or prodrugs thereof, or hydrates thereof, or solvates thereof, and the following compounds include: Defactinib, CEP-28122, CEP-37440, TAE226, PF-562271, PF-431396, VS-4718, PF-573228, BI853520, IN10018, and APG-2449;

alternatively, the FAK inhibitor is a compound represented by formula I, or optical isomers thereof, or tautomers thereof, or salts thereof, or prodrugs thereof, or hydrates thereof, or solvates thereof,

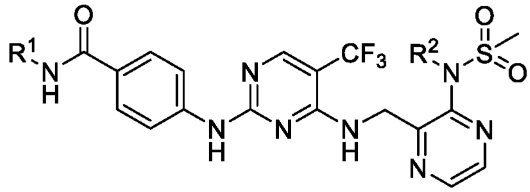

Formula I

wherein, $R^1$ and $R^2$ are each independently selected from the group consisting of hydrogen, methyl, and trideuteromethyl;
alternatively, the FAK inhibitor is the following compounds, or optical isomers thereof, or tautomers thereof, or salts thereof, or prodrugs thereof, or hydrates thereof, or solvates thereof, and the following compounds are

selected from the group consisting of:

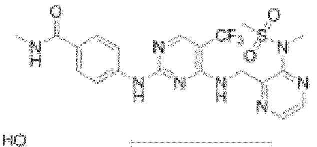

1

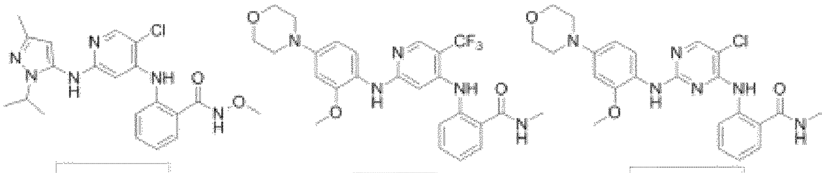

2 3 4

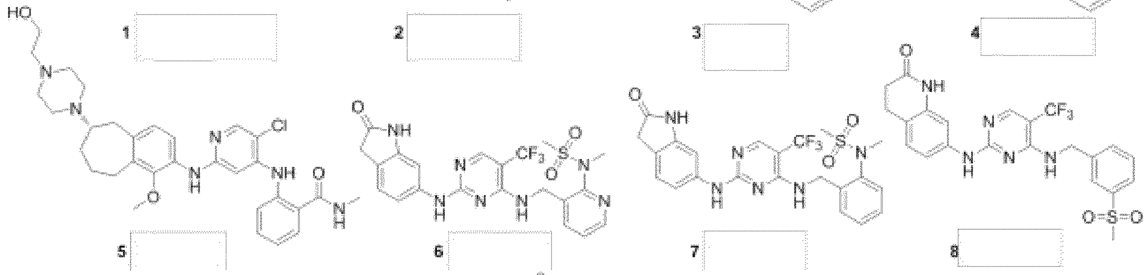

5 6 7 8

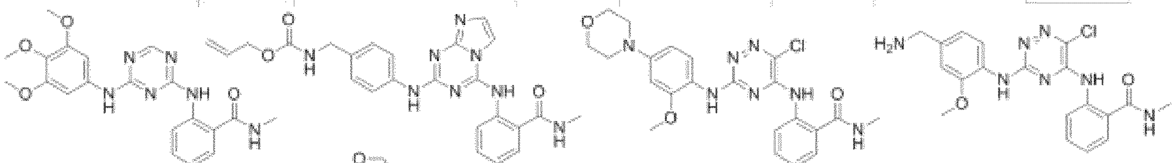

9 10 11 12

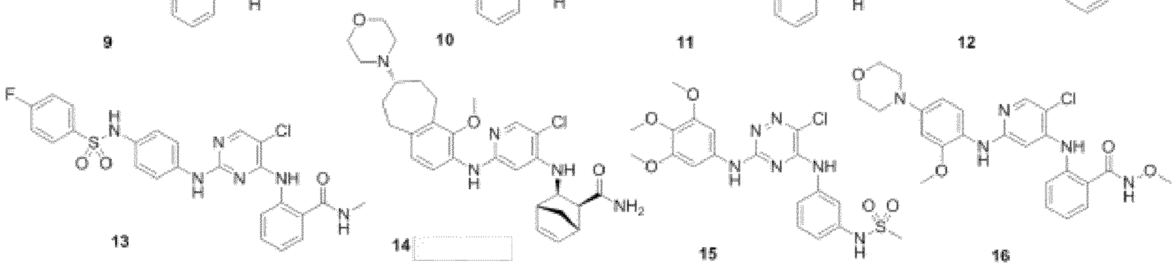

13 14 15 16

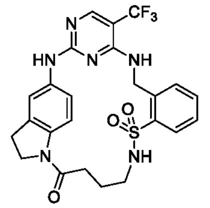

17

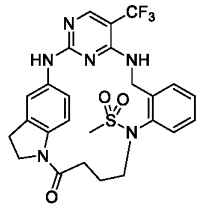

18

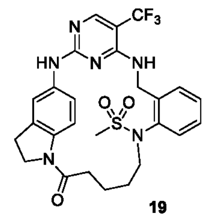

19

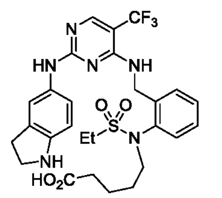

20

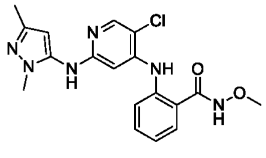

21

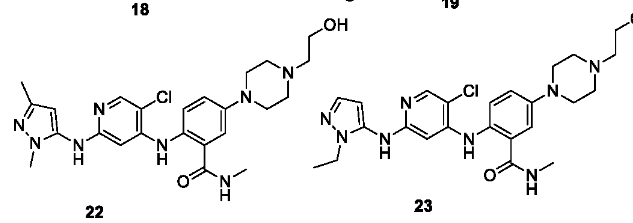

22 23

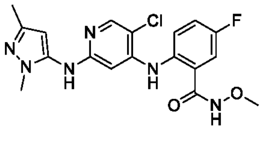

24

;

preferably, the FAK inhibitor is the following compounds, or optical isomers thereof, or tautomers thereof, or salts thereof, or prodrugs thereof, or hydrates thereof, or solvates thereof, and the following compounds include

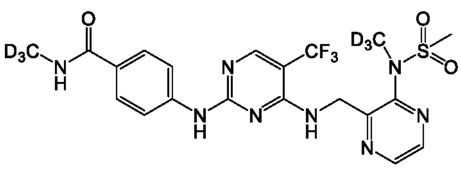

,

Defactinib or IN10018.

**16.** The use according to claim 14, **characterized in that** the CD44 inhibitor is the following compounds, or optical

isomers thereof, or tautomers thereof, or salts thereof, or prodrugs thereof, or hydrates thereof, or solvates thereof, or peptides, or antibodies, and the following compounds or peptides or antibodies include Angstrom6, PEP-1, AMC303, Anti-CD44 mAb H4C4, anti-CD44 mAb HI44a, anti-CD44 mAb IM7, anti-CD44 mAb KM201, RO5429083, RG7356, anti-CD44V6 mAb 2F10, anti-CD44V6 mAb U36, anti-CD44V6 mAb V6B3, anti-CD44V6 mAb VFF18, anti-CD44V6 mAb VFF4, anti-CD44V6, and mAb VFF7;
preferably, the CD44 inhibitor is the following compound, or optical isomers thereof, or tautomers thereof, or salts thereof, or prodrugs thereof, or hydrates thereof, or solvates thereof, and the following compound includes AMC303.

**17.** The use according to any one of claims 14 to 16, **characterized in that** the FAK inhibitor is compound

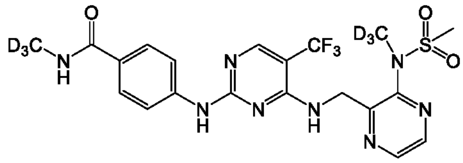
,

or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 0.4-81:1;

alternatively, the FAK inhibitor is Defactinib, or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 0.4-27:1;
alternatively, the FAK inhibitor is IN 10018, or optical isomers thereof, or tautomers thereof, or salts thereof, or prodrugs thereof, or hydrates thereof, or solvates thereof; the CD44 inhibitor is AMC303, or optical isomers thereof, or tautomers thereof, or salts thereof, or prodrugs thereof, or hydrates thereof, or solvates thereof; the molar ratio of FAK inhibitor and CD44 inhibitor is 1:1.

**18.** The use according to any one of claims 14 to 16, **characterized in that** the cancer is solid tumor, mesothelioma, melanoma, prostate cancer, breast cancer, glioblastoma, and brain cancer;

the solid tumors include mesothelioma, pancreatic cancer, soft tissue tumor, metastatic tumor, non-solid cancer, sarcoma, adenocarcinoma, lung cancer, breast cancer, lymphoma, gastrointestinal cancer, urogenital system cancer, prostate cancer, ovarian cancer; the gastrointestinal cancer includes colon cancer, and the urogenital system cancer includes renal, urothelial or testicular tumors; the ovarian cancer includes advanced ovarian cancer;
the mesothelioma includes neurofibroma, renal cancer, lung cancer, small cell lung cancer, non-small cell lung cancer, KRAS-mutant non-small cell lung cancer, liver cancer, thyroid cancer, breast cancer, nervous system tumor, neurilemmoma, meningioma, neuroma, adenoid cystic carcinoma, ependymoma, ependymal tumor, malignant pleural tumor, malignant pleural mesothelioma, triplet tumor, negative breast cancer, non-blood malignant tumor, melanoma, colorectal cancer, leukemia, adenocarcinoma, and solid tumors;
the melanoma includes locally advanced melanoma, locally mutant N-Ras driven melanoma, and metastatic malignant skin melanoma; the colorectal cancer includes metastatic colorectal cancer; the leukemia includes acute myelogenous leukemia; the adenocarcinoma includes adenocarcinoma; the solid tumors include locally advanced solid tumors, metastatic solid tumors, and hepatocellular carcinoma;
the prostate cancer includes castration-resistant prostate cancer and metastatic castration resistant prostate cancer;
the brain cancer includes neuroepithelial tissue tumors, cerebral gliomas, astrocytomas, oligodendrogliomas, ependymal and choroidal plexus tumors, pineal tumors, neurocellular tumors, gangliocytoma, neuroblastoma, poorly differentiated tumors, embryonal tumors, glioblastoma multiform, medulloblastoma, schwannoma, meningioma, malignant lymphoma, cerebrovascular tumors, vascular malformations capillary telangiectases, pituitary tumors, and metastatic tumors.

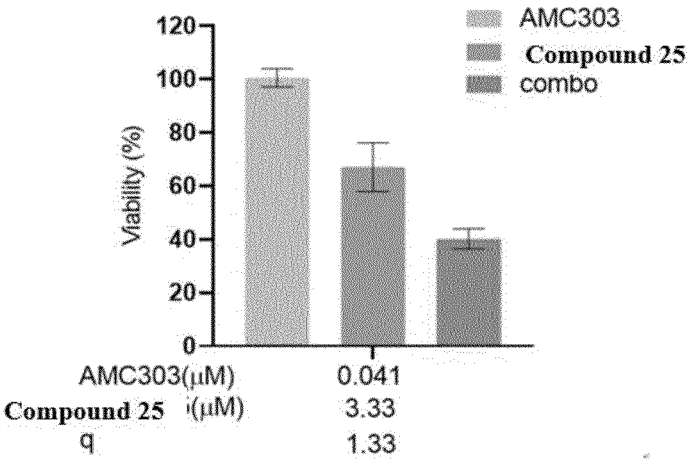


Figure 1

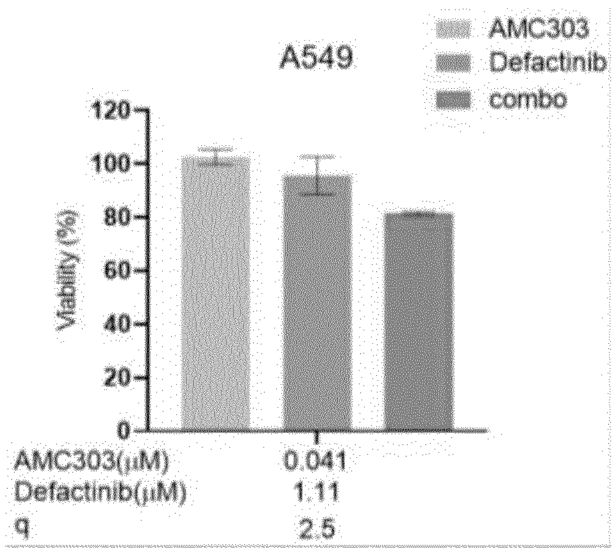


Figure 2

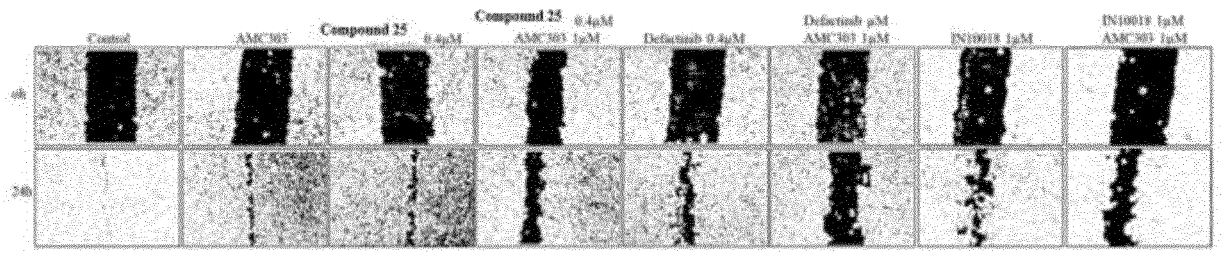


Figure 3

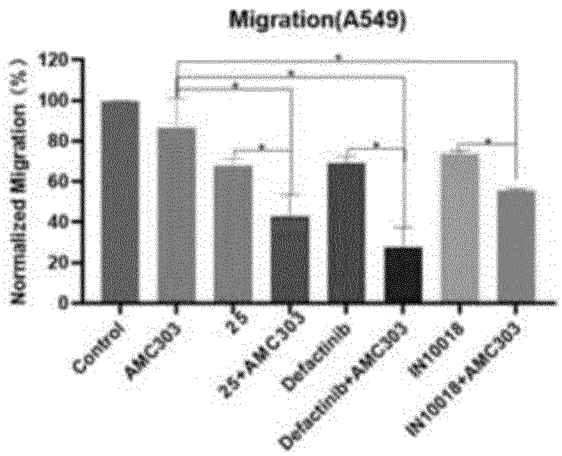


Figure 4

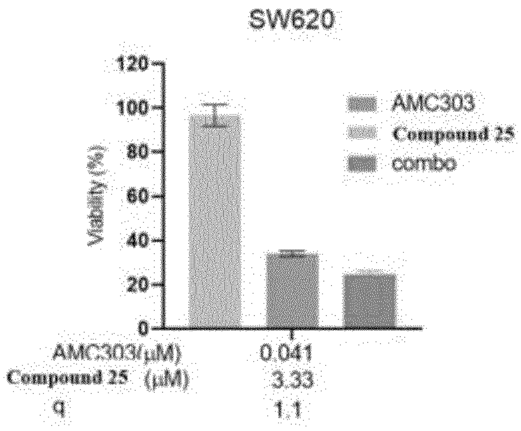


Figure 5

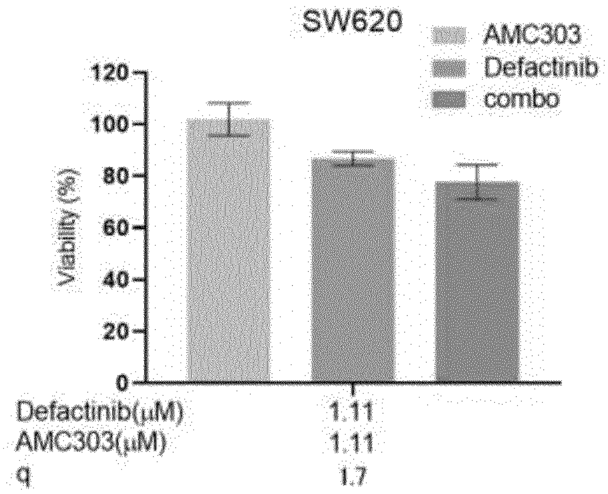


Figure 6

**INTERNATIONAL SEARCH REPORT**

International application No. **PCT/CN2022/094725**

**A. CLASSIFICATION OF SUBJECT MATTER**

A61K 31/506(2006.01)i; A61K 31/4439(2006.01)i; A61K 31/5377(2006.01)i; A61K 31/496(2006.01)i; A61K 31/519(2006.01)i; A61K 31/53(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

DWPI, SIPOABS, WOTXT, EPTXT, USTXT, CNTXT, CATXT, GBTXT, JPTXT, KRABS, CNABS, CNKI, STNext, ISI Web of Science: 细胞表面跨膜糖蛋白, 非受体型酪氨酸蛋白激酶, 癌症, 海创药业, focal adhesion kinase, FAK, CD44, HINOVA, cancer

**C. DOCUMENTS CONSIDERED TO BE RELEVANT**

| Category* | Citation of document, with indication, where appropriate, of the relevant passages | Relevant to claim No. |
|---|---|---|
| X | ZOU, L. et al. "Administration of PLGA Nanoparticles Carrying shRNA against Focal Adhesion Kinase and CD44 Results in Enhanced Antitumor Effects against Ovarian Cancer." *Cancer Gene Therapy,* Vol. 20, 15 March 2013 (2013-03-15), pp. 242-250 | 1-2, 5, 10, 14 |
| Y | ZOU, L. et al. "Administration of PLGA Nanoparticles Carrying shRNA against Focal Adhesion Kinase and CD44 Results in Enhanced Antitumor Effects against Ovarian Cancer." *Cancer Gene Therapy,* Vol. 20, 15 March 2013 (2013-03-15), pp. 242-250 | 3-4, 6-9, 11-13, 15-18 |
| Y | CN 111377871 A (HINOVA PHARMACEUTICALS INC.) 07 July 2020 (2020-07-07) abstract, and claims 1 and 9 | 3, 6-9, 11-13, 15-18 |
| Y | CALVO, E. et al. "First-in-Human, First-in-Class Study of the CD44v6 Inhibitor AMC303 as Monotherapy in Patients with Advanced Epithelial Tumors." *Annals of Oncology,* Vol. 29, No. supplement 8, 31 October 2018 (2018-10-31), abstract | 4, 6-9, 12-13, 16-18 |
| A | CN 106146406 A (SHENZHEN TARGETRX, INC.) 23 November 2016 (2016-11-23) abstract, and claims 1-9 | 1-18 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 August 2022** | **23 August 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

**INTERNATIONAL SEARCH REPORT**

International application No.
**PCT/CN2022/094725**

**C. DOCUMENTS CONSIDERED TO BE RELEVANT**

| Category* | Citation of document, with indication, where appropriate, of the relevant passages | Relevant to claim No. |
|---|---|---|
| A | WO 2008129380 A1 (PFIZER PRODUCTS INC.) 30 October 2008 (2008-10-30)<br>abstract, and claims 1-20 | 1-18 |
| A | WO 2011109678 A1 (ANGSTROM PHARMACEUTICALS, INC.) 09 September 2011 (2011-09-09)<br>abstract, and claims 1-5 | 1-18 |

Form PCT/ISA/210 (second sheet) (January 2015)

INTERNATIONAL SEARCH REPORT

International application No. **PCT/CN2022/094725**

**Box No. III Observations where unity of invention is lacking (Continuation of item 3 of first sheet)**

This International Searching Authority found multiple inventions in this international application, as follows:

[1] Invention 1: the FAK inhibitor in claims 1-2, 5, 10, 14, 17 (all), 3-4, 6-9, 11-13, 15-16, and 18 (in part) is a compound of formula I.

[2] Invention 2: the FAK inhibitor in claims 3-4, 6-9, 11-13, 15-16, and 18 (in part) is defactinib.

[3] Invention 3: the FAK inhibitor in claims 3-4, 6-9, 11-13, 15-16, and 18 (in part) is CEP-28122.

[4] Invention 4: the FAK inhibitor in claims 3-4, 6-9, 11-13, 15-16, and 18 (in part) is CEP-37440.

[5] Invention 5: the FAK inhibitor in claims 3-4, 6-9, 11-13, 15-16, and 18 (in part) is TAE226.

[6] Invention 6: the FAK inhibitor in claims 3-4, 6-9, 11-13, 15-16, and 18 (in part) is PF-562271.

[7] Invention 7: the FAK inhibitor in claims 3-4, 6-9, 11-13, 15-16, and 18 (in part) is PF-431396.

[8] Invention 8: the FAK inhibitor in claims 3-4, 6-9, 11-13, 15-16, and 18 (in part) is VS-4718.

[9] Invention 9: the FAK inhibitor in claims 3-4, 6-9, 11-13, 15-16, and 18 (in part) is PF-573228.

[10] Invention 10: the FAK inhibitor in claims 3-4, 6-9, 11-13, 15-16, and 18 (in part) is BI853520.

[11] Invention 11: the FAK inhibitor in claims 3-4, 6-9, 11-13, 15-16, and 18 (in part) is IN10018.

[12] Invention 12: the FAK inhibitor in claims 3-4, 6-9, 11-13, 15-16, and 18 (in part) is APG-2449.

[13] Inventions 13-36: the FAK inhibitors in claims 3-4, 6-9, 11-13, 15-16, and 18 (in part) relating to inventions 13-36 respectively are compounds 1-24.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-2, 5, 10, 14, 17 (all), 3-4, 6-9, 11-13, 15-16, and 18 (in part)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2022/094725**

| Patent document cited in search report | | | Publication date (day/month/year) | Patent family member(s) | | | Publication date (day/month/year) |
|---|---|---|---|---|---|---|---|
| CN | 111377871 | A | 07 July 2020 | KR | 20210110846 | A | 09 September 2021 |
| | | | | WO | 2020135442 | A1 | 02 July 2020 |
| | | | | BR | 112021012682 | A2 | 08 September 2021 |
| | | | | EP | 3904351 | A1 | 03 November 2021 |
| | | | | JP | 2022515273 | A | 17 February 2022 |
| | | | | CA | 3125058 | A1 | 02 July 2020 |
| | | | | AU | 2019414550 | A1 | 22 July 2021 |
| | | | | US | 2022125788 | A1 | 28 April 2022 |
| CN | 106146406 | A | 23 November 2016 | WO | 2017143842 | A1 | 31 August 2017 |
| WO | 2008129380 | A1 | 30 October 2008 | EC | SP099694 | A | 30 November 2009 |
| | | | | CN | 101678215 | A | 24 March 2010 |
| | | | | PE | 20090434 | A1 | 13 April 2009 |
| | | | | NI | 200900190 | A | 27 January 2010 |
| | | | | AU | 2008240359 | A1 | 30 October 2008 |
| | | | | CO | 6260093 | A2 | 22 March 2011 |
| | | | | HK | 1136985 | A1 | 16 July 2010 |
| | | | | CL | 2008001076 | A1 | 24 October 2008 |
| | | | | MX | 2009011090 | A | 02 November 2009 |
| | | | | US | 2017327485 | A1 | 16 November 2017 |
| | | | | JP | 2010524914 | A | 22 July 2010 |
| | | | | MA | 31319 | B1 | 01 April 2010 |
| | | | | US | 2020002310 | A1 | 02 January 2020 |
| | | | | KR | 20090130248 | A | 21 December 2009 |
| | | | | DO | P2009000248 | A | 15 November 2009 |
| | | | | GT | 200900272 | A | 17 May 2010 |
| | | | | CN | 103951658 | A | 30 July 2014 |
| | | | | DK | 2146779 | T3 | 28 November 2016 |
| | | | | US | 2017001981 | A1 | 05 January 2017 |
| | | | | US | 2009054395 | A1 | 26 February 2009 |
| | | | | US | 2013005964 | A1 | 03 January 2013 |
| | | | | AP | 200905010 | D0 | 31 October 2009 |
| | | | | CU | 20090175 | A7 | 21 September 2011 |
| | | | | NZ | 580372 | A | 12 January 2012 |
| | | | | EA | 200901250 | A1 | 30 April 2010 |
| | | | | UA | 97834 | C2 | 26 March 2012 |
| | | | | UY | 31026 | A1 | 28 November 2008 |
| | | | | US | 2011166120 | A1 | 07 July 2011 |
| | | | | ES | 2593486 | T3 | 09 December 2016 |
| | | | | CA | 2684447 | A1 | 30 October 2008 |
| | | | | US | 2015080368 | A1 | 19 March 2015 |
| | | | | GE | P20125581 | B | 25 July 2012 |
| | | | | EP | 2146779 | A1 | 27 January 2010 |
| | | | | US | 2014100368 | A1 | 10 April 2014 |
| | | | | TN | 2009000428 | A1 | 31 March 2011 |
| | | | | BR | PI0810411 | A2 | 14 October 2014 |
| | | | | US | 2016130250 | A1 | 12 May 2016 |
| | | | | AP | 200905010 | A0 | 31 October 2009 |
| | | | | PA | 8777101 | A1 | 19 November 2008 |
| | | | | HK | 1200450 | A1 | 07 August 2015 |
| | | | | AR | 066125 | A1 | 22 July 2009 |

**INTERNATIONAL SEARCH REPORT**
**Information on patent family members**

International application No.
**PCT/CN2022/094725**

| Patent document cited in search report | Publication date (day/month/year) | Patent family member(s) | Publication date (day/month/year) |
|---|---|---|---|
| | | US 2019047979 A1 | 14 February 2019 |
| | | IL 260560 B | 29 April 2021 |
| | | IL 232511 D0 | 30 June 2014 |
| | | IL 201468 D0 | 31 May 2010 |
| | | TW 200848050 A | 16 December 2008 |
| WO 2011109678 A1 | 09 September 2011 | EP 2542253 A1 | 09 January 2013 |
| | | US 2011217233 A1 | 08 September 2011 |
| | | AU 2011223563 A1 | 01 November 2012 |
| | | CA 2795698 A1 | 09 September 2011 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 201910373081 A


### Non-patent literature cited in the description


- **MERZABAN, J, S et al.** Leukemia: Official journal of the Leukemia Society of America. Leukemia Research Fund, 2016